# EUROPEAN PATENT APPLICATION

(11) **EP 4 194 554 A1**
(43) Date of publication of application: **14.06.2023**
(21) Application number: 21865732.8
(22) Date of filing: 04.08.2021
(51) Int. Cl.: C12N 15/113, A61K 31/713, A61P 35/00

(54) **SIRNA SEQUENCE FOR EFFECTIVELY INHIBITING EXPRESSION OF EPIDERMAL GROWTH FACTOR RECEPTOR**

(30) Priority: 11.09.2020 CN 202010953769
(71) Applicant: Jenkem Technology Co., Ltd. (Beijing), Haidian District Beijing 100192 (CN)
(72) Inventor: WANG, Genyu, Beijing 100192 (CN); LIN, Meina, Beijing 100192 (CN); ZHAO, Xuan, Beijing 100192 (CN)
(74) Representative: HGF
(86) International application number: PCT/CN2021/110432
(87) International publication number: WO 2022/052676

(57) **Abstract**

Provided are an interfering RNA for inhibiting gene expression of an epidermal growth factor receptor (EGFR for short) and an application of the interfering RNA. The interfering RNA is siRNA, comprises nucleotide sequences of GCAACAUGUCGAUGGACUU and/or AAGUCCAUCGACAUGUUGC, can effectively inhibit the gene expression of the EGFR, and provides a new choice for preventing or treating EGFR-related diseases (particularly EGFR-related cancers).

## Description

### TECHNICAL FIELD

The present invention relates to the technical field of molecular biology and biomedicine, and in particular to a siRNA for effectively inhibiting the gene expression of epidermal growth factor receptor (EGFR for short).

### BACKGROUND

Many life activities of vertebrate cells are regulated by extracellular signal molecules. These signals are often transmitted through the cell membrane by some transmembrane receptors, wherein one class of important receptors have protein tyrosine kinase activity itself and belong to the family of receptor protein tyrosine kinases (RPTKs). As a typical member of this family, the epidermal growth factor receptor (EGFR) plays its role via signaling pathways in the cell membrane. Different pathways have different functions, and the same pathway has different functions in different cells or even in the same cell under different conditions.

EGFR is a multifunctional transmembrane protein molecule existing on the cell surface and wide distribution. It has a molecular weight of 170 kD, and it consists of 1186 amino acid residues and is encoded by protooncogene c-erbB1. The human EGFR gene is located in the p13-q22 region of chromosome 7 and is structurally divided into 3 regions: an amino-terminal region extending to the outside of the membrane to recognize and bind to EGF, a single strand α-helical transmembrane region in the middle of the cell membrane, and a carboxyl-terminal region in the cytoplasm having tyrosine kinase activity. The intracellular 542 amino acids can be divided into 3 domains: the juxtamembrane domain (about 50 amino acids) acts as a binding site for negative feedback by PKC (protein kinase C) and erk MAPK (extracellular signal-regulated kinase mitogen-activated protein kinase), and evidence suggests that a motif in this domain may link to the heterotrimeric G protein. Next comes a tyrosine kinase domain (SH1) comprising approximately 250 amino acids; a single 229-amino acid-long carboxyl-terminal tail comprises 5 autophosphorylation motifs. It binds to proteins containing SH2 (src homology domain 2) or PTB (phosphotyrosine binding domain), and at least 3 internalization motifs each comprise a tight turn, a site for transphosphorylation and a site for protein decomposition and degradation. EGFR autophosphorylation motifs are structurally similar and functionally redundant, different from other RPTKs; simple structures and alternate occurrences of complex and redundant motifs represent an archetypal gene.

The ligand of the EGFR binds to the EGFR on the cell membrane, so that the receptor forms a dimer, and the dimeric receptor activates the tyrosine kinase itself. The phosphorylated tyrosine on the receptor binds to a SH2 domain of growth factor receptor-binding protein 2 (Grb2) located on the membrane, and meanwhile SH3 domain of Grb2 binds to guanylate exchange factor SOS (Son of Sevenless). The latter dissociates GDP of small molecule guanylate-binding protein Ras to bind to GTP, thereby activating Ras. The activated Ras further binds to the amino terminal of a serine/threonine protein kinase Raf-1 to activate the Raf-1 by an unknown mechanism. The Raf-1 can phosphorylate two regulatory serines on MEKI/MEK2 MAP kinase/ERK kinase, thereby activating MEK. The MEK is a specific kinase that can phosphorylate serine/threonine and tyrosine and ultimately activate ERK1 and ERK2 with high selectivity. ERKs are proline-directed serine/threonine kinases that can phosphorylate serine/threonine adjacent to the proline. After mitogen stimulation, ERK receives cascade reaction signals from a upstream and can translocate into a human nucleus. Therefore, ERK can not only phosphorylate plasmosin but also phosphorylate some transcription factors in the nucleus, such as c-fos, c-jun, Elk-1, c-myc and ATF2, and the like, thereby participating in the regulation of cell proliferation and differentiation.

The deep research on EGFR signaling has significant meaning in clarifying various physiological processes such as cell growth, development, division, intercellular function synchronization and the like and pathological processes such as cell malignant transformation and the like. EGFR is often found overexpressed in many tumors of humans, and since it is recognized early as a protooncogene transforming the γ-erbB oncogene and most tumorigenesis, EGFR-mediated downstream signals may represent a new approach to combat tumors.

Patent document CN 101671397 B discloses a recombinant immunotoxin targeting the epidermal growth factor receptor. The recombinant immunotoxin is formed by fusion expression of a single chain antibody of EGFR and a toxin protein Gelonin. This technical scheme shows significant effect by utilizing the targeting capacity of the single chain antibody for EGFR high-expression tumors and the specific killing effect on the tumor by the cytotoxic effect of Gelonin.

Patent document CN 103333246 B discloses a method for preparing an antibody drug conjugate that can resist EGFR receptor and inhibit tumor growth and use of the antibody drug conjugate in the treatment of epidermal growth factor receptor antigen-positive cell cancers.

In patent document CN 106661576 A, Stehlik Bio-corporation and Baylor College of Medicine disclose compositions and methods for preparing and using an RNAi capable of reducing expression of two or more genes, comprising: a first RNAi molecule that reduces the expression of a first target gene; a second RNAi molecule that reduces the expression of the first or a second target gene; and optionally a third RNAi molecule that reduces expression of the first, the second, or a third target gene, wherein the RNAi molecules reduce the expression level of, e.g., mutant KRAS, EGFR, PIK3, NCOA3, or ERα1.

Fu Jianhua et al. ("Research progress of EGFR and E-cadherin on tumorigenesis and tumor development and regulation mechanism thereof. The Practical Journal of Cancer. 2008, 23 (1): 107-109) discloses that EGFR overexpression has a certain positive correlation with tumor formation and plays an important role in division and proliferation of tumors.

Patent document CN 102993305 A discloses that EGFR and its coding gene can be used for preparing specific antibody drugs for preventing and treating tumor and other diseases such as inflammation and autoimmune diseases.

RNA interference (RNAi) is a new gene silencing technology developed in recent years. siRNA is an effector molecule of RNAi, and *in vitro* synthesis of siRNA is the latest development of RNA interference technology, especially in the aspect of specifically inhibiting gene expression of mammal cells, which creates a new way for gene therapy.

### SUMMARY

The present invention provides an interfering RNA for inhibiting EGFR expression.

Specifically, the interfering RNA described above comprises a sense strand and an antisense strand complementary to and paring to the sense strand reversely.

Specifically, the interfering RNA described above is selected from siRNA, dsRNA, shRNA, aiRNA, miRNA, and combinations thereof.

In one embodiment of the present invention, the interfering RNA described above is siRNA.

In one embodiment of the present invention, the interfering RNA described above is chemically synthesized.

Specifically, the interfering RNA described above comprises a nucleotide sequence set forth in SEQ ID NO: 1 or consists of a nucleotide sequence set forth in SEQ ID NO: 1.

Specifically, the interfering RNA described above comprises a nucleotide sequence set forth in SEQ ID NO: 2 or consists of a nucleotide sequence set forth in SEQ ID NO: 2.

Specifically, the sense strand of the interfering RNA described above comprises or consists of a nucleotide sequence as follows: 5'-GCAACAUGUCGAUGGACUU-3'.

Specifically, the antisense strand of the interfering RNA described above comprises or consists of a nucleotide sequence as follows: 5'-AAGUCCAUCGACAUGUUGC-3'.

Specifically, the end (e.g., 3' end) of the sense strand and/or the antisense strand of the interfering RNA molecule described above (e.g., siRNA) can be also provided with n over-hangs to increase the activity of the interfering RNA. The over-hangs can be the same or different deoxynucleosides (e.g., deoxythymidine (dT), deoxycytidine (dC), deoxyuridine (dU), etc.), and n is an integer of 1-10 (e.g., 1, 2, 3, 4, 5, 6, 7, 8, 9 or 10), particularly an integer of 2-4; in one embodiment of the present invention, n = 2, and the over-hangs can be dTdT, dTdC, or dUdU, or the like.

Specifically, the 3' ends of the sense strand and the antisense strand of the interfering RNA molecule described above are both provided with the over-hangs dTdT.

Specifically, the sense strand of the interfering RNA described above comprises or consists of a nucleotide sequence as follows: 5'-GCAACAUGUCGAUGGACUU dTdT-3'.

Specifically, the antisense strand of the interfering RNA described above comprises or consists of a nucleotide sequence as follows: 5'-AAGUCCAUCGACAUGUUGC dTdT-3'.

Specifically, the interfering RNA molecule described above can also comprise at least one modified nucleotide, and a modified interfering RNA has better properties, such as higher stability, lower immune stimulation and the like, than a corresponding unmodified one.

The present invention also provides a delivery system for the interfering RNA described above, which comprises the interfering RNA described above and a vector.

Specifically, the vector described above can be any vector suitable for delivering the interfering RNA of the present invention described above to a target tissue or a target cell, such as those disclosed in the prior art (e.g., Chen Zhonghua, Zhu Desheng, Li Jun, Huang Zhanqin, "Advances in non-viral vector research of siRNA". Chinese Pharmacological Bulletin. 2015, 31 (7): 910-4; Wang Rui, Qu Bingnan, Yang Jing. "Advances in research of siRNA-carrying nano preparation". China Pharmacy 2017, 28 (31): 4452-4455).

Specifically, the vector described above is a viral vector, specifically such as a lentivirus, a retrovirus, an adenovirus, a herpes simplex virus, or the like.

Specifically, the vector described above is a non-viral vector, specifically such as liposome, polymer, polypeptide, antibody, aptamer, etc. or combinations thereof, wherein the weight ratio of the interfering RNA described above to the non-viral vector can be 1:1-50 (e.g., 1:1, 1:5, 1:10, 1:15, 1:20, 1:25, 1:30, 1:35, 1:40, 1:45, 1:50, etc.).

Specifically, the liposome described above can be a cationic liposome (e.g., lipofectamine series of Invitrogenn, 1,2-dioleoyl-3-trimethylammonium propane (DOTAP)), a neutral ion liposome (e.g., dioleoyl phosphatidylcholine (DOPC), cholesterol, etc.), an anion liposome (e.g., dioleoyl phosphatidylglycerol (DOPG), dioleoyl phosphatidylethanolamine (DOPE), etc.), or mixtures thereof.

Specifically, the polymer described above can be a synthetic polymer (e.g., polyethyleneimine, cyclodextrin, etc.) or a natural polymer (e.g., chitosan, atelocollagen, etc.) or mixtures thereof. Specifically, the polypeptide described above can be a cell penetrating peptide (CPP) (e.g., protamine, Tat peptide, transportan peptide, penetratin peptide, oligo-arginine peptide, etc.). Specifically, the antibody described above can be a single chain antibody (e.g., scFv-tp, scFv-9R, etc.).

The present invention also provides a pharmaceutical composition, which comprises the interfering RNA or the delivery system thereof described above, and a pharmaceutically acceptable excipient. The present invention also provides use of the interfering RNA or the delivery system thereof or the pharmaceutical composition described above in preparing a medicament for preventing and/or treating EGFR-related diseases.

The present invention also provides use of the interfering RNA or the delivery system thereof described above in designing a medicament for preventing and/or treating EGFR-related diseases. The present invention also provides the interfering RNA or the delivery system thereof or the pharmaceutical composition described above for use in inhibiting EGFR gene expression in living cells.

The present invention also provides a method for inhibiting EGFR expression in a subject in need thereof, which comprises administering to the subject a therapeutically effective amount of the interfering RNA or the delivery system thereof or the pharmaceutical composition of the present invention described above.

The present invention also provides a method for preventing and/or treating EGFR-related diseases, which comprises administering to a subject a therapeutically effective amount of the interfering RNA or the delivery system thereof or the pharmaceutical composition of the present invention described above.

Specifically, the diseases described above include, but are not limited to, cancer, inflammation, and the like.

Specifically, the cancer described above includes, but is not limited to, lung cancer (e.g., non-small cell lung cancer), liver cancer, esophageal cancer, leukemia, cervical cancer, colorectal cancer, pancreatic cancer, renal cancer, bladder cancer, breast cancer, prostate cancer, gastric cancer, oral epithelial cancer, ovarian cancer, head and neck cancer, brain tumor, glioblastoma, and the like. The present invention also provides a method for introducing the interfering RNA of the present invention described above into a cell, which comprises contacting the cell with the delivery system of the interfering RNA.

Specifically, the cell described above is *in vivo* of a subject.

Specifically, contacting the cell with the delivery system of the interfering RNA described above is administering the delivery system of the interfering RNA to the subject *in vivo* via a systemic route or a local route to contact the cell.

The inventors of the present invention obtain an interfering RNA (e.g., siRNA) via design and synthesis, which can effectively inhibit EGFR expression, and provides a new option for preventing or treating EGFR-related diseases (particularly, EGFR-related cancers).

### BRIEF DESCRIPTION OF THE DRAWINGS

The accompanying drawings, which constitute a part of the present application, are used to provide a further understanding of the present application, and the illustrative examples and description thereof are intended to explain the present application but not to limit the present application.
FIG. 1 shows a mass spectrum of a sense strand of EGFR siRNA.
FIG. 2 shows a mass spectrum of an antisense strand of EGFR siRNA.
FIG. 3 shows expression level of EGFR mRNA in cells.

### DETAILED DESCRIPTION

Unless otherwise defined, all scientific and technical terms used herein have the same meaning as commonly understood by those skilled in the art to which the present invention relates.

The term "interfering RNA", "RNAi" or "interfering RNA sequence" as used herein includes a single strand RNA (e.g., mature miRNA, ssRNAi oligonucleotide, or ssDNAi oligonucleotide) or a double strand RNA (i.e., duplex RNA, e.g., siRNA, dsRNA, shRNA, aiRNA, or pre-miRNA) which can reduce or inhibit expression of a target gene or sequence (e.g., by mediating degradation and inhibiting translation of mRNA complementary to the interfering RNA in sequence) when the interfering RNA and the target gene or sequence are in the same cell. The interfering RNA thus refers to a single strand RNA complementary to the target mRNA in sequence or a double strand RNA formed from two complementary strands or a single self-complementary strand. Specifically, the interfering RNA molecule is chemically synthesized.

The interfering RNA includes "small interfering RNAs" or "siRNAs", and each strand of a siRNA molecule comprises nucleotides of about 15 to about 60 in length (e.g., nucleotides of about 15-60, 15-50, 15-40, 15-30, 15-25, or 19-25 in length, or nucleotides of 15, 16, 17, 18, 19, 20, 21, 22, 23, 24, or 25 in length). In a specific embodiment, the siRNA is chemically synthesized. The siRNA molecule of the present invention can silence expression of a target sequence *in vitro* and/or *in vivo.* In other embodiments, the siRNA comprises at least one modified nucleotide. For example, the siRNA comprises one, two, three, four, five, six, seven, eight, nine, ten, or more modified nucleotides in the double strand region.

As used herein, the term "dsRNA" or "pre-RNAi molecule" is intended to include any precursor molecule that is processed *in vivo* by an endonuclease to produce an active siRNA.

As used herein, the term "small hairpin RNA", "short hairpin RNA" or "shRNA" includes short RNA sequences producing tight hairpin turns that can be used to silence gene expression via RNA interference. The shRNA hairpin structure can be lysed by a cellular machinery to siRNA. Typically, a microRNA (miRNA) is a single strand RNA molecule regulating gene expression with about 21-23 nucleotides in length.

As used herein, the term "therapeutically effective amount" refers to an amount of a subject compound that will elicit the biological or medical response in a tissue, system or subject that is being sought by a researcher, a veterinarian, a medical doctor or other clinicians. The term "therapeutically effective amount" includes the amount of an active ingredient as follows: when administered, it is sufficient to prevent progression of one or more signs or symptoms of a disorder or disease treated, or to some extent, ameliorate one or more signs or symptoms of a disorder or disease treated. The therapeutically effective amount will vary with active ingredients, the disease to be treated and its severity, as well as subject's age, weight, sex, etc.

In the present invention, the subject can be a mammal, e.g., human, monkey, dog, rabbit, mouse, rat, etc.

It should be noted that the detailed description as follows is exemplary and is intended to provide further explanation of the present application.

### Example 1 Synthesis Method of siRNA

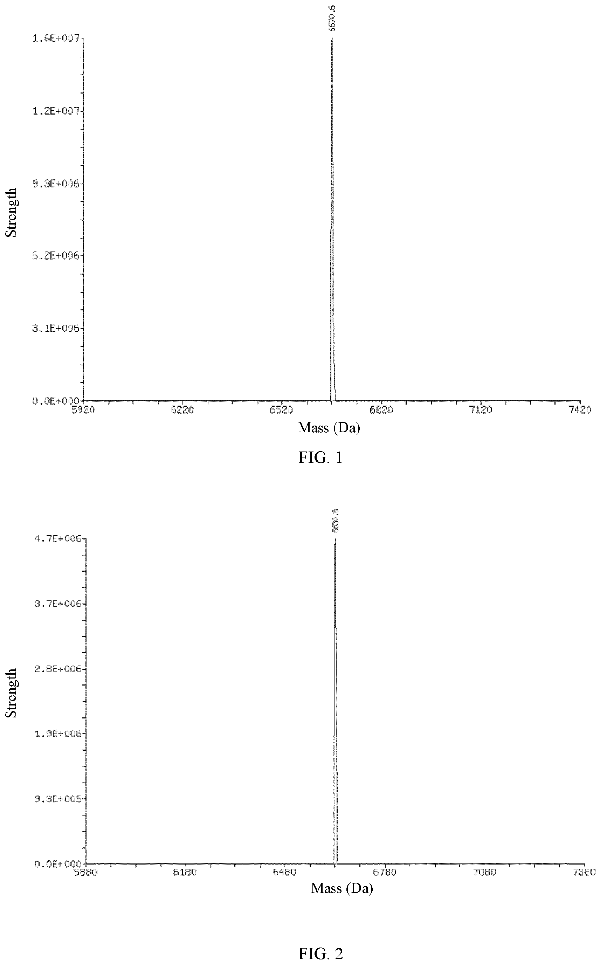

The oligonucleotide comprising 2'-hydroxyl ribonucleotide in the present invention was synthesized according to the theoretical yield of 1 µmol. The weighed 1 µmol of universal solid phase support 3'-cholesterol-modified CPG (Chemgenes product) and the 2'-O-TBDMS-protected RNA phosphoramidite monomer were dissolved in anhydrous acetonitrile solution to reach a concentration of 0.2 M. A solution of 5-ethylthio-1H-tetrazole (Chemgenes product) in acetonitrile was prepared and used as an activator (0.25 M), a 0.02 M solution of iodine in pyridine/water was prepared and used as an oxidant, and a 3% solution of trichloroacetic acid in dichloromethane was used as a deprotection reagent. These reagents were placed at corresponding specified positions for reagents in an ABI 394 DNA/RNA automatic synthesizer. A synthesis program was set and a base sequence of the specified oligonucleotide was input before starting cyclic oligonucleotide synthesis, wherein the coupling time of each step was 6 min, and the coupling time of corresponding L and S monomers of galactose ligand was 10-20 min. After automatic circulation, the oligonucleotide solid phase synthesis was completed. The CPG was blown dry with dry nitrogen and transferred to a 5 mL EP tube, followed by addition of 2 mL of ammonia/ethanol solution (3/1). The mixture was heated at 55°C for 16-18 h. The mixture was centrifuged at 10000 rpm for 10 min, the supernatant was collected, and a white colloidal solid was obtained after strong ammonia/ethanol solution was drained off. The solid was dissolved in 200 µL of 1 M TBAF THF solution, and the mixture was oscillated at room temperature for 20 h. Then 0.5 mL of 1 M Tris-HCl buffer (pH 7.4) was added, and the resulting mixture was oscillated at room temperature for 15 min. Then it was dried in a centrifugal drier to 1/2 original volume to remove THF. The solution was extracted twice with 0.5 mL of chloroform, and then 1 mL of 0.1 M TEAA loading fluid was added. The mixed solution was poured into a solid phase extraction column, and mass spectrometric detection analysis was completed on the HTCS LC-MS system (Novatia). The mass spectrometric identification results are shown in FIG. 1 (sense strand) and FIG. 2 (antisense strand). Nucleic acid molecular weights were calculated by normalization with Promass software after primary scanning. Two single strands were each synthesized through the method described above, and after being identified as correct by mass spectrometry, the two single strands were mixed at an equal molar ratio and annealed into a double strand, namely the siRNA sequence.

The sense strand sequence of the siRNA was: 5'-GCAACAUGUCGAUGGACUU dTdT-3';

The antisense strand sequence of the siRNA was: 5'-AAGUCCAUCGACAUGUUGC dTdT-3'.

### Example 2 Inhibiting Effect of EGFR siRNA

### 1. Cell culturing

### Cell name: 293T

a) 293T cells were conventionally cultured at 37°C and 5% CO₂;
b) 5 µL of siRNA (or siRNA NC with a concentration of 20 µM) was diluted by 50 µL of OPTI-MEM medium, and 3 µL of Lipofectamine RNAiMAX transfection reagent was diluted by 50 µL of OPTI-MEM medium. The two solutions were then mixed, shaken gently, and left to stand for 15 min; in addition, Mock and blank control groups were set;
c) the mixed solution described above was added at 108 µL/well;
d) 293T cells in the logarithmic growth phase were taken and seeded into a 12-well plate at 1.2×10⁵ cells/well and 892 µL/well, and the final total volume of each well was 1000 µL; the transfection concentration of siRNA (or siRNA NC) was 100 nM;
e) the 12-well plate was removed from the incubator at 37°C and 5% CO₂ after transfection for 48 h to extract RNA for subsequent detection.

### 2. RNA extraction

a) Trizol lysis: the cell culture fluid was thoroughly removed and 1 mL of TrizolTM Reagent was added. The cells were then pipetted 3-5 times with a pipette for full lysis and left to stand at room temperature for 3-5 min;
b) 0.2 volume (0.2 mL/1 mL Trizol) of chloroform was added, and the mixture was vortexed for 15 s and left to stand at room temperature for 5 min;
c) the mixture was centrifuged at 12000 rpm and 4°C for 15 min; after liquid separation, the upper aqueous phase (the volume of the aqueous phase accounting for about 60% of the volume of Magzol) was carefully pipetted into a new 1.5 mL centrifuge tube;
d) about 0.6 mL (the same volume as the supernatant) of isopropanol was added; the mixture was well mixed by allowing the tube to be repeatedly upside down, and then was left to precipitate at - 20°C for more than 1 h;
e) the mixture was centrifuged at 12000 rpm and 4°C for 30 min; white precipitate was found at the bottom of the tube, and supernatant was removed;
f) 1 mL of 75% ethanol was added, and the mixture was gently pipetted to allow the precipitate to float; the mixture was centrifuged at 12000 rpm and 4°C for 5 min;
g) the step f was repeated;
h) centrifugation was carried out for a short time after removing supernatant, and a 10 µL pipette was used to thoroughly remove the liquid; the cover of the centrifuge tube was opened for drying, and then an appropriate amount of RNase-free H₂O was added to dissolve the precipitate when it was dried to translucent state.
i) quality control of RNA: RNA content was detected by Nanodrop and RNA integrity was detected by 1% agarose gel electrophoresis.

### 3. Q-PCR detecting procedure

### 3.1 Reverse transcription of RNA

a) Total RNA extracted from the sample was used as a template, and a reaction system was established as follows:

**Table 1 Reaction system**

| Reagent | Amount |
|---|---|
| 5 × RT Buffer | 4µL |
| RTase Mix | 2µL |
| RT primer (oligodT,10µM) | 1µL |
| RNA template | 1µg |
| RNase-free H₂O | Supplement to 20 µL |
| Total | 20µL |

b) the system was well mixed and centrifuged to allow the liquid to be all at the bottom of the tube; the reverse transcription was performed at 42°C for 60 min and 72°C for 10 min, and the product was the cDNA template.

### 3.2 Quantitative PCR

a) A reaction system was established according to the following table:

**Table 2 Reaction system**

| Reagent | Amount |
|---|---|
| 2 × SYBR Green Mix | 10µL |
| Forward primer (10µM) | 0.4µL |
| Reverse primer (10µM) | 0.4µL |
| cDNA | 2µL |
| RNase-free H₂O | 7.2µL |
| Total | 20µL |

The sequences of the EGFR primer and the endogenous reference gene GAPDH primer are shown in Table 3.

**Table 3 Primer sequence**

| Name of primer | Sequence 5'-- 3' |
|---|---|
| GAPDHF | TCTGACTTCAACAGCGACAC |
| GAPDHR | GCCAAATTCGTTGTCATACC |
| EGFRF | TTTGGTGCCACCTGCGTGAA |
| EGFRR | GCCCTTCGCACTTCTTACACTT |

b) Real-time PCR amplification was performed according to the procedure as follows: Pre-denaturation at 95°C for 10 min, followed by the cycle as follows
*95°C for 10 s
60°C for 20 s
70°C for 10 s
Plate reading
Return to * for 40 cycles in total
Plot a melting curve: read the plate every 0.5°C and then stop for 5 s between 70°C and 95°C.

### 4. Inhibiting effect

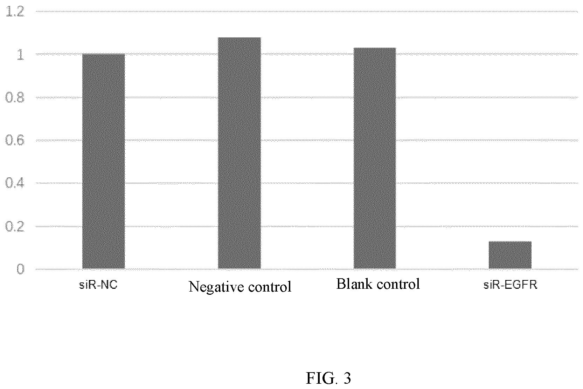

The relative expression level of EGFR mRNA was calculated by ΔΔCt with GAPDH as an endogenous reference gene. Compared with the NC siRNA transfection, the EGFR siRNA has an inhibition rate of 87% for EGFR mRNA. The mRNA expression levels of each group of cells are shown in Table 4 and FIG.3.

**Table 4 mRNA expression levels**

| Treatment | Expression |
|---|---|
| siR-NC | 1 |
| Negative control | 1.08 |
| Blank control | 1.03 |
| siR-EGFR | 0.13 |

The above description is only for the purpose of illustrating the preferred examples of the present invention, and is not intended to limit the scope of the present invention. Any modifications, equivalents and the like made without departing from the spirit and principle of the present invention shall fall in the protection scope of the present invention.

The foregoing examples and methods described herein can vary based on the abilities, experience, and preferences of those skilled in the art.

The certain order in which the steps of the method are listed in the present invention does not constitute any limitation on the order of the steps of the method.

## Claims

1. An interfering RNA, comprising a nucleotide sequence set forth in SEQ ID NO: 1 and/or SEQ ID NO: 2.

2. The interfering RNA according to claim 1, wherein the interfering RNA is selected from siRNA, dsRNA, shRNA, aiRNA, miRNA, and combinations thereof.

3. The interfering RNA according to claim 1, wherein the interfering RNA is siRNA.

4. The interfering RNA according to claim 3, wherein a sense strand of the interfering RNA comprises a nucleotide sequence as follows: 5'-GCAACAUGUCGAUGGACUU-3'; and/or
an antisense strand of the interfering RNA comprises a nucleotide sequence as follows: 5'-AAGUCCAUCGACAUGUUGC-3' .

5. The interfering RNA according to claim 1, wherein the interfering RNA also comprises an over-hang;
preferably, the number of the over-hangs is 1-10, more preferably 2-4;
preferably, the over-hang is deoxynucleoside;
preferably, the over-hang is located at the 3' end of the sense strand and/or antisense strand of the interfering RNA.

6. The interfering RNA according to claim 5, wherein the over-hang is dTdT, dTdC or dUdU.

7. The interfering RNA according to claim 5, wherein a sense strand of the interfering RNA comprises a nucleotide sequence as follows: 5'-GCAACAUGUCGAUGGACUU dTdT-3'; and/or, an antisense strand of the interfering RNA comprises a nucleotide sequence as follows: 5'-AAGUCCAUCGACAUGUUGC dTdT-3'.

8. The interfering RNA according to any one of claims 1-7, wherein the interfering RNA is chemically synthesized.

9. A delivery system for the interfering RNA according to any one of claims 1-8, comprising the interfering RNA according to any one of claims 1-8 and a vector;
preferably, the vector is a viral vector or a non-viral vector.

10. A pharmaceutical composition, comprising the interfering RNA according to any one of claims 1-8 or the delivery system according to claim 9, and a pharmaceutically acceptable excipient.

11. Use of the interfering RNA according to any one of claims 1-8, the delivery system according to claim 9 or the pharmaceutical composition according to claim 10 in preparing a medicament for preventing and/or treating epidermal growth factor receptor-related diseases;
preferably, the diseases are cancer, inflammation.
